# EUROPÄISCHE PATENTANMELDUNG

(11) **EP 2 489 272 A1**
(43) Veröffentlichungstag der Anmeldung: **22.08.2012**
(21) Anmeldenummer: 12000758.8
(22) Anmeldetag: 06.02.2012
(51) Int. Cl.: A21D 6/00, A23L 1/176, A61K 8/06, A61K 8/97

(54) **Verwendung von Mehlextraktgranulaten als Emulgator**

(30) Priorität: 16.02.2011 DE 102011011416; 26.08.2011 DE 102011111660
(71) Anmelder: Eduard Walter KG, 67459 Böhl-Iggelheim (DE)
(72) Erfinder: Berizzi, Hans-Peter, 67459 Böhl-Iggelheim (DE)
(74) Vertreter: Wagner, Jutta

(57) **Zusammenfassung**

Die vorliegende Erfindung betrifft die Verwendung von Mehlextraktgranulaten, erhältlich indem von gemahlenem Getreide eine mit Protein angereicherte Mehlfraktion durch Windsichtung abgetrennt und mit Wasser, welches 0,1 bis 2 Gew.-% Säure enthält, granuliert wird, als Emulgator, sowie emulgierende Mischungen enthaltend die Mehlextraktgranulate und ein Verfahren zu ihrer Herstellung.

## Beschreibung

Die vorliegende Erfindung betrifft die Verwendung von Mehlextraktgranulaten als Emulgator, sowie emulgierende Mischungen enthaltend Mehlextraktgranulate und ein Verfahren zu ihrer Herstellung.

Emulgatoren sind Hilfsstoffe, die dazu dienen, zwei nicht miteinander mischbare Flüssigkeiten, wie zum Beispiel Öl und Wasser, zu einem fein verteilten Gemisch, der sogenannten Emulsion, zu vermengen und zu stabilisieren. Ähnliches gilt für die Aufmischung von festen, nicht löslichen Stoffen in einer Flüssigkeit, um eine sogenannte Suspension zu stabilisieren. Emulgatoren sind in vielen Bereichen wichtig, unter anderem in der Lebensmittel- und Kosmetikindustrie. Gerade in diesen Bereichen gibt es unzählige Vorschläge, jedoch besteht noch immer Bedarf an solchen Substanzen bzw. Mischungen, die als natürlich qualifiziert werden können. Darunter fallen vor allem möglichst naturnah hergestellte pflanzliche Produkte. Auch in dieser Hinsicht sind bereits diverse Systeme bekannt.

Bereits 1931 beschrieb die DE 516363 einen Zusatz von Pektin zu Dauerbackwarenteig, um die Wasserbindung zu verbessern. Die DD 266 960 offenbart ein Verfahren zur Gewinnung von multifunktionellen, löslichen, protein-und pentosanhaltigen Produkten aus zerkleinertem Roggen. Diese Produkte sollen eine Schaumbildung und Emulgierung in Lebensmitteln bewirken und die Schäume bzw. Emulsionen auch stabilisieren. Gemäß DE 44 36 989 lassen sich isolierte, wasserlösliche Bestandteile von Roggen als Ei-Ersatz in Dressings und Cremes für die Patisserie sowie zur Stabilisierung von Schlagsahne nutzen. Die DE 10 2006 019 241 betrifft ein Verfahren zur Herstellung einer Proteine und Polysaccharide enthaltenden Öl- oder Flüssigfett-in-Wasser Emulsion, bei der das Öl oder Fett mit einem Biopolymergemisch bestehend aus einer wässrigen Phase mit Proteinen und einem Polysaccharid ohne Säurezugabe bei einem Protein:Polysaccharid-Verhältnis von 1:0,25 bis 1:2,0 emulgiert und anschließend der pH-Wert durch Zugabe einer Säure gesenkt wird. Als Protein wird Molkenprotein und als Polysaccharid Pektin und/oder Na-Carboxymethylcellulose eingesetzt. Aus der DE 10 2009 019 551 ist ein Verfahren zur Herstellung eines Nahrungsmittels bekannt, bei welchem 0,2 bis 75 % einer Öl-in-Wasser-Emulsion enthaltend Protein, polares Polysaccharid und Fett bzw. Öl sowie ggfs. Polyol, Aroma, und/oder Säure zu einer Nahrungsmittelbasis gegeben werden. Die Emulsion soll eine Trockenmasse von 5 bis 60 % und eine Partikelgröße der Fett bzw. Öltropfen im Verteilungsmaximum von maximal 10 µm haben.

Schließlich sind aus EP 713 365 Roggenmehle bekannt, bei denen durch eine Windsichtung des aus den Körnern gemahlenen Mehls eine Roggenmehlfraktion mit erhöhtem Protein- und Ballaststoffgehalt und eine mit erniedrigtem Protein- und Ballaststoffgehalt erzeugt werden. Diese Roggenmehlfraktionen sollen dann mit "natürlichem" Mehl gemischt zur Herstellung von Backwaren dienen. Ein ähnliches Verfahren ist aus DE 102 48 160 bekannt, wobei aus Weizenmehl durch Windsichtung eine proteinreiche Fraktion abgetrennt, mit Wasser granuliert und einem Backmehl beigemischt wird.

Überraschend wurde nun gefunden, dass sich die gemäß DE 102 48 160 erhaltenen Mehlextraktgranulate nicht nur als Zusatz für Backmehl eignen, sondern auch hervorragend als Emulgator für andere Lebensmittel und bei der Herstellung von Kosmetika.

Die vorliegende Erfindung betrifft daher die Verwendung von Mehlextraktgranulaten als Emulgator für Kosmetika und für Nahrungsmittel mit Ausnahme von Backwaren. Die Erfindung betrifft außerdem eine emulgierende Mischung enthaltend Mehlextraktgranulate und Wasser und Verfahren zu deren Herstellung.

Die erfindungsgemäß verwendeten Emulgatoren zeichnen sich dadurch aus, dass sie im wesentlichen durch physikalische Prozesse aus dem Pflanzenprodukt Getreidemehl gewonnen werden, und somit als weitgehend natürlich zu bezeichnen sind. Es erfolgen keine Extraktionen mit Wasser oder Lösemitteln, die Abtrennung erfolgt allein über die Korngröße bzw. Dichte der Teilchen bei der Windsichtung des Mehls. Die erfindungsgemäß verwendeten Emulgatoren erlauben die Einstellung von Viskosität, Konsistenz und Wasserbindung bei Nahrungsmitteln und Kosmetika und zeigen eine gute Emulsionsbildung und - stabilisierung sowie Schaumbildung und -stabilisierung.

Zur Herstellung der Emulgatoren erfolgt zunächst ein Mahlen des Getreides zu Mehl. Als Getreide wird bevorzugt Weizen eingesetzt, es sind aber auch Roggen, Hafer, Gerste, Mais, etc. geeignet. Sodann wird das Mehl mittels Windsichtung in zwei, vorzugsweise in drei oder gewünschtenfalls in noch mehr Fraktionen aufgeteilt. Bei der üblichen Aufteilung in drei Fraktionen erhält man die Bestandteile kalibriertes Mehl, schwach proteinhaltiges Mehl und stark proteinhaltiges Mehl.

Erfindungsgemäß sind die mit Protein angereicherten Mehlfraktionen von Interesse, insbesondere das stark proteinhaltige Mehl, das bei drei Fraktionen von 5 bis 30 %, typischerweise etwa 10 bis 15 %, des Mehls ausmacht und bei dem etwa 80 % der Teilchen kleiner als 90 µm sind. Mittlere Teilchengrößen liegen häufig im Bereich von 20 bis 60 µm.

Die gewünschte(n) protein-angereicherte(n) Mehlfraktion(en) werden dann, beispielsweise in der Wirbelschicht mit Wasser oder nach Suspendierung mit einem Walzentrockner, granuliert. Mit Granulierung sind vorliegend Verfahren gemeint, die aus der pulverigen Mehlfraktion partikelförmige Granulate von beliebiger Form erzeugen. Bevorzugt werden keine Bindemittel zugefügt.

Für die Wirbelschichtgranulierung werden ca. 30 Gew.-% Wasser bezogen auf die Mehlfraktion eingesprüht. Wichtig ist, dass bei einer Temperatur von Raumtemperatur bis maximal 40 °C granuliert wird, um thermische Veränderungen der Proteine und anderer Inhaltsstoffe auszuschließen. Die Verweilzeit in der Wirbelschicht-Granulierung beträgt typischerweise etwa 20 bis 60 Minuten, eine mehrstufige, kontinuierliche Granulierung wird daher bevorzugt.

Für die Walzentrocknung wird die Mehlfraktion in Wasser suspendiert und auf die langsam drehende, z.B. mit Dampf beheizte Walze aufgetragen. Bei einem Walzentrockner reicht typischerweise eine ¾ Drehung, das getrocknete Produkt wird dann mit einem Messer abgeschält und das so erhaltene Granulat, z.B. mit einem Förderband, abtransportiert. Es ist für die Verwendung als emulgierende Mischung von Vorteil, dass bei der Walzentrockung die Mehlfraktion auf Temepraturen von mindestnes 80 °C, vorzugsweise auf etwa 83 °C erhitzt wird.

Gewünschtenfalls wird die Granulatteilchengröße durch Sieben eingestellt und/oder durch Zerkleinern reduziert. Zur Zerkleinerung eignen sich beispielsweise Hammermühlen.

Bei der Granulierung wird eine Säure beigefügt, z.B. dem Wasser zugemischt. Geeignet sind vor allem zum Einsatz in Lebensmitteln zugelassene Säuren, beispielsweise Essigsäure und/oder Milchsäure. Üblicherweise erfolgt die Zugabe in einer Menge von 0,1 bis 2 Gew.-%, vorzugsweise von 0,2 bis 1 Gew.-% bezogen auf das Mehl.

Auch andere Stoffe können bei der Granulierung zugefügt werden. Dadurch lassen sie sich sehr gleichmäßig und homogen in dem Nahrungsmittel oder der Kosmetik verteilen. Wasserlösliche Stoffe werden typischerweise in dem zur Granulierung verwendeten Wasser gelöst. Ebenso können Zusätze trocken oder in Form einer Lösung/Dispersion/Suspension zugefügt werden. Daraus resultierende zusätzliche Wassermengen werden bei der Wasserzugabe berücksichtigt.

In einer bevorzugten Ausführungsform werden der proteinreichen Mehlfraktion bei der Granulierung zusätzlich Nichtgetreideprotein, wie Leguminosen- oder Molkenprotein, und/oder Nicht-Stärke-Polysaccharid, wie Pektin oder Carboxymethylcellulose, zugefügt.

Als Produkt der Windsichtung und Granulierung erhält man das Mehlextraktgranulat, welches in Kosmetika oder Nahrungsmitteln als Emulgator verwendet werden kann. Der Proteingehalt liegt bei Weizen typischerweise im Bereich von 16 bis 24 Gew.-%. Eine Anreicherung der Proteine durch die Windsichtung um 30 bis 200 %, typischerweise um 50 bis 100 % wird angestrebt. Das Mehlextraktgranulat hat eine Korngröße von 100 bis 400 µm, vorzugsweise von 250 bis 300 µm. Die Feuchte beträgt typischerweise von 6 bis 12 Gew.-% und liegt damit noch unter derjenigen von unbehandeltem Mehl. Das Mehlextraktgranulat lässt sich aufgrund seiner Struktur gut handhaben, es kann ohne weiteres 12 Monate gelagert werden.

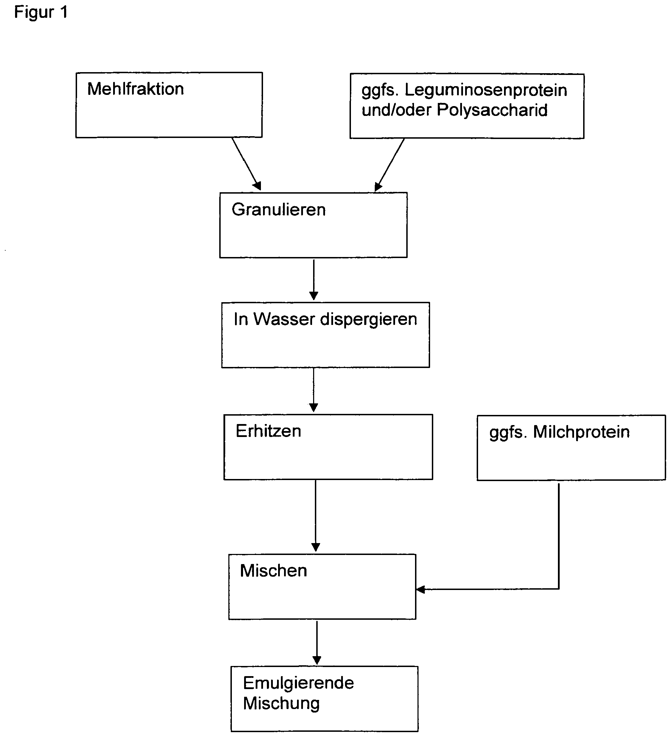

Die unter der erfindungsgemäßen Verwendung des Mehlextraktgranulates hergestellte Emulsion sollte, wenn es sich um in der Wirbelschicht granulierte Mehlfraktion handelt, bei der Herstellung für 5 bis 20 Minuten einer Temperatur von 60 bis 90 °C, vorzugsweise von 75 bis 85 °C ausgesetzt werden, um die Stärke zu verkleistern. Die Erhitzung muss erfolgen, bevor hitzeempfindliche Bestandteile zugefügt werden, ggfs. stellt die Dispergierung des Mehlextraktgranulats in Wasser und Erhitzung dieser wässrigen Dispersion den ersten Schritt bei der Herstellung des Kosmetikums oder Lebensmittels dar. Das Verfahren ist in Figur 1 veranschaulicht. Für die mittels Walzentrocknung granulierte Mehlfraktion kann die Erhitzung entfallen, wenn auf der Walze Temperaturen von mindestens 80 °C, vorzugsweise von etwa 83 °C erreicht werden.

Zur Herstellung der erfindungsgemäßen emulgierenden Mischung wird die granulierte, protein angereicherte Mehlfraktion im Gewichtsverhältnis von 1:2 bis 1:6, vorzugsweise von 1:3 bis 1:5 und besonders bevorzugt von etwa 1:4 in Wasser dispergiert.

In einer bevorzugten Ausführungsform wird der emulgierenden Mischung eines oder mehrere Nicht-Stärke-Polysaccharide zugemischt. Die Menge liegt im Bereich von 0,1 bis 20 Gew.-%, vorzugsweise 1 bis 15 Gew.-%, besonders bevorzugt 8 bis 12 Gew.-%, bezogen auf das Trockengewicht der Mischung. Bevorzugte Polysaccharide sind Pektin, z.B. Citrus- oder Apfelpektin, und Carboxymethylcellulose, z.B. Natriumcarboxymethylcellulose (Na-CMC). Besonders bevorzugt wird eine wässrige Pektinlösung zugemischt. Die Zumischung verbessert in Emulsionen die Wasserbindung und Gefrier-Tau-Stabilität sowie die Emulsionsstabilität (Aufrahm- und Koaleszenzstabilität).

In einer weiteren, bevorzugten Ausführungsform werden der emulgierenden Mischung von 0,1 bis 30 Gew.-%, vorzugsweise von 1 bis 15 Gew.-%, besonders bevorzugt von 8 bis 12 Gew.-%, bezogen auf das Trockengewicht der Mischung, Nichtgetreideprotein beigefügt. Besonders geeignet sind beispielsweise Milch- bzw. Molkenprotein und Leguminosenprotein, insbesondere Leguminosenprotein, wie Lupinen-, Erbsen- oder Sojabohnenprotein. Der Zusatz des Nichtgetreideproteins erhöht die Stabilität der Grenzschicht an den Öltropfen und verhindert somit eine Tropfenaggregation in der Emulsion. Dadurch wird die Ölaufnahme verbessert und die Emulsion insgesamt stabiler.

Je nach Anwendung können die Wirkungen der beiden Zusätze auch kombiniert werden. In einer ganz besonders bevorzugten Ausführungsform werden daher der emulgierenden Mischung sowohl Nichtgetreideprotein, vorzugsweise Leguminosenprotein, zugefügt als auch eine wässrige Lösung von Nicht-Stärkepolysacchariden, vorzugsweise Pektin und/oder Carboxymethylcellulose zugemischt.

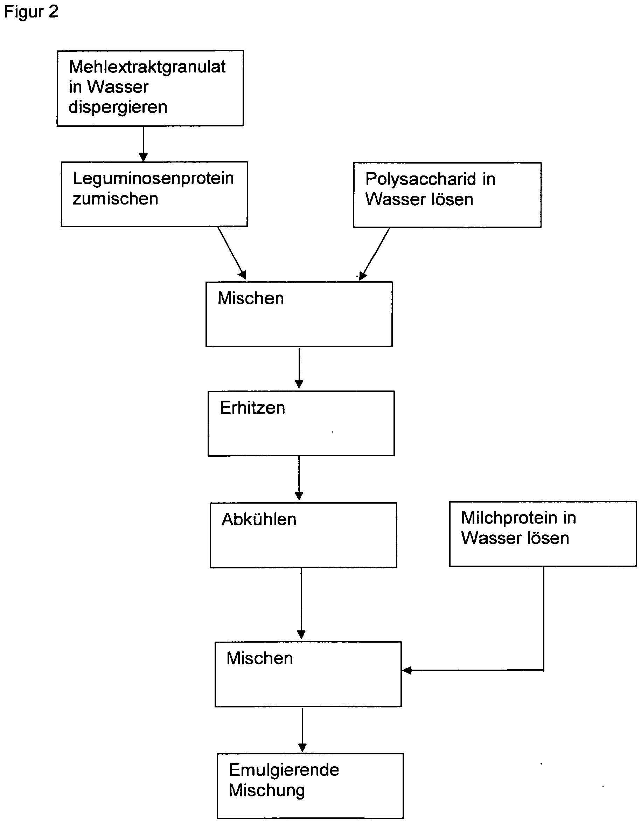

Die emulgierende Mischung kann bevorzugt hergestellt werden, indem zunächst 50 bis 100 g Mehlextraktgranulat in 300 g Wasser dispergiert werden. In die Dispersion rührt man gewünschtenfalls 5 bis 15 g Nichtgetreideprotein ein. Soll ein Polysaccharid zugemischt werden, erfolgt dies vorzugsweise in Form einer wässrigen Lösung, die das bzw. die Polysaccharid(e) in einer Menge von 1 bis 5 g/100g, vorzugsweise von 3 bis 4 g/100g enthält. Die Lösung des (der) Polysaccharids(Polysaccharide) wird im Gewichtsverhältnis 1:0,5 bis 1:2, vorzugsweise1:0,75 bis 1:1,5 und besonders bevorzugt etwa 1:1 mit der Mehlextraktgranulatdispersion vermischt. Die Mischung kann, falls bei der Granulierung keine ausreichendenTemperaturen erreicht wurden, 1 bis 100, vorzugsweise 5 bis 20 Minuten auf 60 bis 90 °C, vorzugsweise 75 bis 85 °C erhitzt werden, um die Stärke zu verkleistern. Sofern Milchprotein als Nichtgetreideprotein zum Einsatz kommt, wird dessen wässrige Lösung bevorzugt erst nach einem Erhitzen, z.B. nach dem Abkühlen auf unter 55 °C, zugemischt. Das Verfahren ist in Figur 2 veranschaulicht. Die emulgierende Mischung kann in der Form, wie sie erhalten wird, verwendet werden.

Die erfindungsgemäße, emulgierende Mischung eignet sich hervorragend als Emulgator und Stabilisator für Kosmetika und für Nahrungsmittel, wie Dressings, Cremefüllungen, Saucen, Suppen, Dessertspeisen und vieles mehr. Die viskositätsgebenden, emulgierenden und schaumbildenden Eigenschaften lassen sich durch die Zusätze Nichtgetreideprotein und Polysaccharid sowie die Mengenverhältnisse einstellen.

Das Mehlextraktgranulat und die emulgierende Mischung enthalten vorzugsweise bezogen auf die Trockenmasse entweder nur proteinreiche Mehlfraktion, oder 60 bis 99,9 Gew.-% proteinreiche Mehlfraktion gemischt mit 0,1 bis 20 Gew.-% Nichtgetreideprotein und/oder 0,1 bis 20 Gew.-% Nicht-Stärke-Polysaccharid.

Die Menge Mehlextraktgranulat oder emulgierende Mischung wird vorzugsweise so gewählt, dass dessen Trockengewicht 0,5 bis 15 Gew.-% des Lebensmittels bzw. Kosmetikums ausmacht. Eine Flüssigdispersion zur Verwendung in Lebensmitteln kann z.B. 3 bis 13 Gewichtsteile Mehlextraktgranulat mit 1 bis 55 Teilen Öl und/oder Fett und 42 bis 96 Teilen Wasser enthalten. Eine Flüssigdispersion zur Verwendung in Kosmetika kann z.B. 3 bis 13 Gewichtsteile Mehlextraktgranulat mit 1 bis 55 Teilen Öl und/oder Fett und 42 bis 96 Teilen Wasser enthalten.

Gegenüber dem Mehlextraktgranulat ohne weiteren Protein- oder Polysaccharid-Zusatz weisen mit der besonders bevorzugten emulgierenden Mischung hergestellte Emulsionen (z. B. mit 5 % bis 30 % Öl) eine bessere Wasserbindung und Lagerstabilität hinsichtlich einer Phasentrennung und eine hohe Gefrier-Tau-Stabilität und Hitzestabilität auf.

Ein Vergleich mit nach DE 102 48 160 angereichertem Roggenmehl (21,1 % Protein, 25,2 % Pentosan, 47,6 % Stärke) zeigte, dass bei der erfindungsgemäßen, zusätzlichen Anreicherung mit Leguminosen- oder Milchprotein und Polysacchariden (Pektin, Na-CMC) eine Emulsion mit 5 bis 55 % Ölanteil eine bessere Wasserbindung und Lagerstabilität hinsichtlich Phasentrennung und eine hohe Gefrier-Tau-Stabilität sowie Hitzestabilität erreicht wird. Weiterhin war bei der zusätzlich angereicherten und erhitzen Roggenmehlprobe ein geringerer Roggengeschmack spürbar.

Da Molkenprotein gegenüber den Leguminosenproteinen temperaturempfindlicher ist und mit dem Molkenprotein höher konzentrierte wässrige Lösungen hergestellt werden können, wird gelöstes Molkenprotein der erfindungsgemäßen emulgierenden Mischung nach der Stärkeverkleisterung durch Erhitzen und Abkühlung bei etwa 50 °C oder darunter zugesetzt.

Die mit der Erfindung erzielten Vorteile bestehen darin, dass durch Windsichtung mit Protein aufkonzentriertes und durch Säurebehandlung bei der Granulierung agglomeriertes Getreidemehl (Weizen, Roggen usw.) mit den für die Emulgier-, Konsistenz- und Schaumgebung wichtigen Inhaltsstoffen (lösliche und unlösliche Proteine, Nichtstärke-Polysaccharide) aufkonzentriert ist und zusätzlich mit weiteren Proteinen und/oder Polysacchariden angereichert wird.

Die im Mehlextraktgranulat anwesenden Stärkepartikel werden bei Zusatz von Polysacchariden wie Pektin oder Na-CMC infolge höherer Viskosität während der zusätzlichen Erhitzung vor einer Sedimentation geschützt. Bei einer Nacherhitzung der Suspension erfolgt somit kein Absatz eines Stärkegels (insbesondere bei Weizenmehlextraktgranulat).

Es wurde weiterhin überraschend festgestellt, dass nach Zugabe der Pektinlösung zur emulgierenden Mischung die Nichtgetreideproteine bei Hitzebelastung (z. B. 80 °C, 10 bis 20 Minuten) nicht denaturieren und damit hergestellte Emulsionen eine hohe Hitzestabilität aufweisen (keine Phasentrennung, kein Ölabsatz, bei bis zu 60 Minuten bei 90 °C).

Ebenfalls wurde überraschend festgestellt, dass die emulgierenden Mischungen, die Nichtgetreideproteine und Polysaccharide enthalten, in wässriger Lösung bei pH-Senkung nicht zur Bildung unlöslicher Aggregate führen und somit die grenzflächenaktiven Eigenschaften (Emulgiereigenschaften, Schaumbildungseigenschaften) des eingesetzten Mehlextraktgranulates nicht nur voll erhalten bleiben, sondern noch verstärkt werden.

Die erfindungsgemäßen emulgierenden Mischungen, die Milch- oder Leguminosenprotein und Pektin oder Na-CMC enthalten, unterstützen hervorragend die Emulsionsbildung und führen zu hochviskosen Emulsionen mit Partikelgrößen x₅₀ 2,0 bis 3,0 µm beim Dispergieren mittels Rotor-Stator-Prinzip (Zahnkranzdispergiergeräten) und gleichmäßiger Einzeltropfenverteilung ohne Partikelaggregation bei pH-Senkung.

Die positiven Protein-Polysaccharid-Wechselwirkungen (Viskositätsgebung, Konsistenzgebung) bleiben in den erfindungsgemäßen emulgierenden Mischungen erhalten und können bei der Einarbeitung in Lebensmittelsysteme oder Kosmetika ihre Wirkung hinsichtlich Emulsionsbildung, Viskositätsgebung und Schaumbildung voll entfalten.

Bei geringerem Anteil an Nichtstärke-Polysaccharid ergibt sich eine stärkere Schaumbildung bei Anwesenheit schaumbildender Proteine, da die Interaktionen zwischen Proteinen und den ionischen Polysacchariden eher eine Schaumstabilisierung unterstützen.

Die zu erzielende Viskositätsgebung, Emulgiereigenschaft und Schaumbildung bzw. Schaumstabilisierung kann über eine Zugabe unterschiedlicher Anteile an Nichtgetreide-Protein sowie Polysaccharid eingestellt werden.

Die Erfindung soll anhand der folgenden Beispiele erläutert werden, ohne jedoch auf die speziell beschriebenen Ausführungsformen beschränkt zu sein. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

### Beispiel1 1

70 g Weizenmehlextraktgranulat, das nach DE 102 45 60 aus Mehl durch Windsichtung und Wirbelschicht-Granulierung mit 0,2 Gew.-% Milch- und Essigsäure enthaltendem Wasser erhalten wurde, wurde mit 10 g Erbsenproteinisolat (Pisane C9, Provital, Belgien) trocken vermischt. Die Trockenmischung wurde in 426 g Leitungswasser (11 mg Ca/l) mindestens 30 min bei 50 °C unter Rühren dispergiert (Lösung A). Parallel dazu wurden 10 g hochverestertes Citruspektin (CU 201, Herbstreit & Fox KG) in 334 g Leitungswasser bei 70 °C unter Rühren zu einer klaren Lösung aufgelöst (Lösung B). Anschließend werden Lösungen A und B unter Rühren zusammengefügt und bei 80 bis 82 °C (Temperatur der Lösung) etwa 10 min erhitzt. Die Viskosität der Lösung bzw. Stärkedispersion stieg hierbei durch die Verkleisterung der Stärkepartikel an. Diese hochviskose, wasserhaltige Mischung kann nach dem Abkühlen direkt oder nach Wasserentzug (z.B. Gefriertrocknung) für die Lebensmittel- oder Kosmetikaherstellung verwendet werden.

Wird dieses Gemisch direkt zur Emulsionsbildung eingesetzt, erfolgt nach dem Abkühlen auf etwa 50 bis 55 °C das Eindispergieren von 150 g flüssigem Fett oder nach dem Abkühlen auf etwa 20 bis 30 °C das Eindispergieren von Pflanzenöl (z. B. Sonnenblumenöl). Wird die durch Scherdispergien hergestellte Voremulsion höheren Scherkräften mittels Zahnkranzdispergiergerät unterworfen, beträgt der erreichte mittlere Partikeldurchmesser x₅₀ 2,5 µm. Die helle Emulsion ist geschmacksneutral, weist eine hohe Viskosität und gute Cremigkeit auf, ist nach Erhitzung (90 °C, 60 min) phasenstabil, nach 2x Einfrieren bei -18 °C gefrier-tau-stabil und verdünnt (1:1) ebenfalls phasenstabil.

Die Emulsion (pH 4,8) wurde durch Zusatz von Citronensäurelösung im pH-Wert auf pH 4,2 gesenkt. Die Emulsionsstabilität blieb unverändert.

### Beispiel 2

Aus 70 g Roggenmehlextraktgranulat wurde analog Beispiel 1 eine emulgierende Mischung hergestellt. Diese Mischung kann nach dem Abkühlen direkt für die Lebensmittelherstellung verwendet oder mit reduziertem Wasseranteil Lebensmitteln oder Kosmetika zur Qualitätseinstellung untergemischt werden.

Wird dieses Gemisch direkt zur Emulsionsbildung eingesetzt, erfolgt das Eindispergieren wie in Beispiel 1. Die Emulsion mit einem leichten Roggengeschmack weist eine hohe Viskosität und Cremigkeit auf, ist nach 60 min Erhitzung auf 90 °C phasenstabil, nach 2x Einfrieren bei - 18 °C gefrier-tau-stabil und verdünnt (1:1) ebenfalls phasenstabil.

Nach einer zusätzlichen Erhitzung ist der Roggengeschmack nicht mehr spürbar. Erfolgt die Erhitzung nach pH-Absenkung durch Citronensäure (Absenkung von pH 5,6 auf pH 4,8) ist die Emulsion ebenfalls stabil, es ist kein säuerlicher Geschmack spürbar, die Emulsion ist geschmacksneutral.

### Beispiel 3

70 g Weizenmehlextraktgranulat, wie in Beispiel 1 hergestellt, wurden mit 10 g Sojaproteinisolat (Soypro 900 IP, KERRY, Irland) trocken vermischt und in 426 g Leitungswasser mindestens 30 min bei 50 °C unter Rühren dispergiert (Lösung A). Parallel dazu wurden 10 g Na-Carboxymethylcellulose (WALOCEL CRT 1.000 GA, DOW Europe, Bomlitz) in 334 g Leitungswasser bei 70 °C unter Rühren zur klaren Lösung aufgelöst (Lösung B). Anschließend wurden die Lösungen A und B unter Rühren zusammengefügt und bei 80 bis 82 °C (Temperatur der Lösung) 10 min erhitzt. Die Viskosität der Dispersion stieg hierbei durch die Verkleisterung der Stärkepartikel an. Dies hochviskose wasserhaltige Mischung kann nach dem Abkühlen direkt für die Lebensmittel- oder Kosmetikaherstellung verwendet oder mit reduziertem Wasseranteil Lebensmitteln bzw. Kosmetika zur Qualitätseinstellung untergemischt werden.

Zur Emulsionsbildung wurde in das abgekühlte Gemisch 150 g Sonnenblumenöl dispergiert und anschließend mittels Rotor-Stator-Systems (Ultra-Turrax T 25) bei 24 000 U/min feindispergiert. Die erhaltene feincremige helle Emulsion ist geschmacksneutral, weist eine mittlere Partikelgröße x₅₀ von 2 µm auf und ist nach zusätzlicher Erhitzung (90 °C, 60 min) phasenstabil (unverdünnt und 1:1 verdünnt). Nach zweimaligem Einfrieren bei - 18 °C ist diese ebenfalls stabil und behält die Cremigkeit.

Die Emulsion (pH 6,4) wurde durch Zusatz von Citronensäurelösung im pH-Wert auf pH 5,5 gesenkt. Die Emulsion wird etwas dickcremiger, die Stabilität bleibt unverändert.

### Beispiel 4

70 g Roggenmehl, wie in Beispiel 1 durch Windsichtung angereichert, wurden mit 350 g Leitungswasser mindestens 30 min bei 50 °C unter Rühren dispergiert (Lösung A). Parallel dazu wurden 10 g hochverestertes Citruspektin (CU 201, Herbstreit & Fox KG) in 334 g Leitungswasser bei 70 °C unter Rühren gelöst (Lösung B) und 10 g Molkenproteinkonzentrat (Bayolan P 50 Bio, Bayerische Milchindustrie eG) in 76 g Wasser bei 30 °C gelöst (Lösung C). Die Dispersion A wird mit dem gelösten Citruspektin (Lösung B) zusammengefügt und bei 80 bis 82 °C 10 min (Temperatur der Lösung) erhitzt. Die Viskosität der Lösung bzw. Dispersion steigt hierbei durch die Verkleisterung der Stärkepartikel und Quellung der Pentosane an. Nach dem Abkühlen des Gemischs auf etwa 50 °C wurde Lösung C eingerührt. Diese hochviskose, wasserhaltige Mischung kann nach dem Abkühlen zur Emulsionsbildung wie in Beispiel 1 bis 3 eingesetzt werden. Weiterhin ist der direkte Einsatz in der Lebensmittel- oder Kosmetikaherstellung oder nach Trocknung der Einsatz als hochfunktioneller Emulgator zur Qualitätseinstellung möglich.

### Beispiel 5

Die Eigenschaften der Mischungen von Beispiel 1 bis 4 und von weiteren Mischungen wurden an Öl-in Wasser-Emulsionen mit 15 % Ölphase, den angegebenen Mengen Mehlfraktion, Protein, Polysaccharid und Rest Wasser untersucht. Die Ergebnisse sind in Tabelle 1 für Roggenmehlextraktgranulate und in Tabelle 2 für Weizenmehlextraktgranulate zusammengefasst. Die Gefrierstabilität wird durch zweimaliges Einfrieren der Emulsion bei - 18 °C bestimmt und mit + für sehr stabil, (+) für geringen Ölabsatz und - für eine deutlich sichtbare Ölphase bewertet. Die Phasenstabilität wird durch Lagerung der 1:1 verdünnten Emulsion bei 18 °C für 7 Tage bestimmt und mit + für stabil oder - bei Wasserabsatz oder Entmischung bewertet. Die Phasenstabilität wurde analog auch nach Erhitzung der 1:1 verdünnten Emulsion auf 90 °C für 60 min und dann 7 tägige Lagerung bei 18 °C bestimmt, es ergaben sich keine Unterschiede zu der nicht erhitzten Emulsion.

**Tabelle 1: Roggenmehlextraktgranulate**

| Nr. | Getreide: | Citruspektin | Molkeprotein | Erbsen protein | Sojaprotein | pH-Wert | Gefrierstab. | Phasenstab.1:1 |
|---|---|---|---|---|---|---|---|---|
| 1* | 9% | - | - | - | - | 5,8 | - | - |
| 2 | 9% | - | - | - | - | 5,6 | - | - |
| 3 | 7% | 1 % | - | - | - | 4,6 | + | + |
| 4 | 7% | - | - | - | 1 % | 5,7 | - | + |
| 5 | 7% | 1 % | - | - | 1 % | 4,9 | + | - |
| 6 | 7% | - | - | 1 % | - | 5,6 | - | - |
| 7 | 7% | 1 % | - | 1 % | - | 5,2 | + | (+) |
| 8 | 7% | 1 % | 1 % | - | - | 4,2 | + | + |

| | | | | | | | | |
|---|---|---|---|---|---|---|---|---|
| * ohne Erhitzen zur Stärkeverkleisterung | | | | | | | | |

**Tabelle 2: Weizenmehlextraktgranulate**

| Nr. | Getreide: | Citruspektin | Apfelpektin | Na-CMC | Erbsenprotein | Sojaprotein | pH-Wert | Gefrierstab. | Phasenstab. 1:1 |
|---|---|---|---|---|---|---|---|---|---|
| 9* | 9% | - | - | - | - | - | 5,7 | - | - |
| 10 | 9% | - | - | - | - | - | 5,6 | - | - |
| 11 | 7% | - | 1 % | - | - | - | 4,3 | - | + |
| 12 | 7% | 1 % | - | - | - | - | 4,1 | (+) | + |
| 13 | 7% | - | - | - | 1 % | - | 6,1 | - | + |
| 14 | 7% | 1 % | | - | 1 % | - | 4,8 | (+) | + |
| 15 | 7% | - | - | - | - | 1 % | 6,0 | - | + |
| 16 | 7% | 1 % | - | - | - | 1 % | 5,2 | + | + |
| 17 | 7% | - | - | 1 % | - | 1 % | 5,5 | - | + |

| | | | | | | | | | |
|---|---|---|---|---|---|---|---|---|---|
| * ohne Erhitzen zur Stärkeverkleisterung | | | | | | | | | |

Die Ergebnisse in Tabellen 1 und 2 verdeutlichen die positiven Effekte der Mehlextraktgranulate auf die Emulsionseigenschaften.

Während die Mehlextraktgranulate ohne zusätzliche Hydrokolloide nach der Emulsionsbildung (in Emulsionen mit 5 bis 30 % Sonnenblumenöl getestet) unerhitzt und nach der Erhitzung 1:1 verdünnt noch verbesserungsfähige Phasenstabilitäten aufweisen, führt die Anreicherung mit Citruspektin zu einer guten Phasenstabilität der unerhitzten und erhitzten Emulsionen und zu guter Gefrier-Tau-Stabilität (Nr. 3, Tab 1 und Nr. 12, Tab. 2).

Wird dem Weizenmehlextraktgranulat Leguminosenprotein zugesetzt (Soja- oder Erbsenprotein), ist die Phasenstabilität ebenfalls sehr gut, eine ausreichende Gefrier-Tau-Stabilität (kein Ölabsatz nach dem Auftauen der Emulsion) ist nicht gegeben (Proben 13 und 15, Tab. 2). Diese Proben und Roggenmehlextraktgranulat mit Erbsen- und Sojaprotein (Proben 4 und 6, Tab. 1) weisen dagegen auch eine Schaumbildung auf. Der bessere Lufteinschlag wurde an praktischen Lebensmittelsystemen getestet.

Enthalten erfindungsgemäße Mischungen mit Roggen- und Weizenmehlextraktgranulaten zugleich Nichtgetreideproteine und Nichtstärke-Polysaccharide, so ist eine sehr gute Phasenstabilität der verdünnten Emulsion und der erhitzen Emulsion (60 min, 90 °C) gegeben (Probe 7 in Tab. 1 und 14, 16 und 17 in Tab. 2).

Eine gute Gefrier-Tau-Stabilität (Emulsion mit 15 und 30 % Ölgehalt) wird bei Einsatz von Roggenmehlextraktgranulat mit Citruspektin und Sojaproteinisolat (Probe 5), Roggenmehlextraktgranulat mit Citruspektin und Erbsenproteinisolat (Probe 7), Roggenmehlextraktgranulat mit Citruspektin und Molkenprotein (Probe 8), und Weizenmehlextraktgranulat mit Citruspektin und Sojaproteinisolat (Probe 15) (siehe Tab. 1 und 2)..

### Beispiel 6

Weizenmehl wurde mittels Windsichtung in drei Fraktionen aufgeteilt. Die Fraktion stark proteinhaltiges Mehl wurde mit 0,2 Gew.-% Essig- und Milchsäure enthaltendem im Gewichtsverhältnis von etwa 1 Teil Mehlfraktion pro 2 Teile Wasser suspendiert. Die Granulierung erfolgte mit einem Einwalzentrockner, der mit Dampf von 5 bar beheizt wurde und drei Auftragswalzen hatte. Die Suspension der Mehlfraktion wurde mithilfe eines Fahrwagens mittig auf die erste Auftragswalze aufgetragen. Nach einer ¾ Drehung war das Produkt angetrocknet und wurde mit einem Messer abgeschabt. Mit einem Förderband wurde das Mehlextraktgranulat abtransportiert und mittels Hammermühle zerkleinert.

Das so hergestellte Mehlextraktgranulat besitzt den Vorteil einer im Vergleich zur Wirbelschichtrockung einfacheren Herstellung. Außerdem wird durch die Walzentrocknung die Keimzahl deutlich erniedrigt, Verringerungen von 100.000 Keimen pro g auf < 100 Keime pro g wurden beobachtet, was im Hinblick auf die Anwendung in Kosmetika und Lebensmitteln sehr wichtig ist.

Die Walzentrocknung erlaubt vorteilhaft, die Stärke direkt bei der Granulierung zu verkleistern, so dass ein Erhitzungsschritt bei der Verwendung des Mehlextraktgranulats entfallen kann, bzw. nur auf solche Temperaturen erhitzt werden muss, die aus anderen Gründen, z.B. für die Suspendierung/Lösung oder Emulgierung, nötig sind. Als besonders günstig hat sich eine Walzentrocknung erwiesen, bei der das Produkt auf über 80 °C, insbesondere auf mindestens 83 °C erhitzt wird. Temperaturen deutlich über 90 °C bringen keinen weiteren Vorteil, verursachen aber höhere Kosten und sind daher unerwünscht.

## Patentansprüche

1. Verwendung von Mehlextraktgranulat, erhältlich indem von gemahlenem Getreide eine mit Protein angereicherte Mehlfraktion durch Windsichtung abgetrennt und mit Wasser, welches 0,1 bis 2 Gew.-% Säure enthält, granuliert wird, als Emulgator für Kosmetika oder Lebensmittel mit Ausnahme von Backmehl.

2. Verwendung gemäß Anspruch 1, **dadurch gekennzeichnet, dass** für Kosmetika oder Lebensmittel 0,5 bis 15 Gew.-% Mehlextraktgranulat verwendet werden.

3. Verwendung gemäß Anspruch 1 oder 2, **dadurch gekennzeichnet, dass** der Mehlfraktion bei der Granulierung Nichtgetreideprotein und/oder Nicht-Stärke-Polysaccharid beigefügt werden.

4. Verwendung gemäß Anspruch 1, 2 oder 3, **dadurch gekennzeichnet, dass** in dem Mehlextraktgranulat auf 60 bis 99,9 Gew.-% Mehlfraktion 0,1 bis 20 Gew.-% Nichtgetreideprotein und/oder 0,1 bis 20 Gew.-% Nicht-Stärke-Polysaccharid enthalten sind.

5. Verwendung gemäß Anspruch 3 oder 4, **dadurch gekennzeichnet, dass** das Nichtgetreideprotein ausgewählt ist aus Leguminosenprotein, Molkenprotein und Gemischen davon.

6. Verwendung gemäß Anspruch 3, 4 oder 5, **dadurch gekennzeichnet, dass** das Nicht-Stärke-Polysaccharid ausgewählt ist unter Pektin, Carboxymethylcellulose und Gemischen davon.

7. Emulgierende Mischung enthaltend Mehlextraktgranulat, erhältlich indem von gemahlenem Getreide eine mit Protein angereicherte Mehlfraktion durch Windsichtung abgetrennt und mit Wasser, welches 0,1 bis 2 Gew.-% Säure enthält, granuliert wird, und Wasser.

8. Emulgierende Mischung enthaltend Mehlextraktgranulat, erhältlich indem von gemahlenem Getreide eine mit Protein angereicherte Mehlfraktion durch Windsichtung abgetrennt und mit Wasser, welches 0,1 bis 2 Gew.-% Säure enthält, granuliert wird, und mindestens ein Nichtgetreideprotein und/oder mindestens ein Nicht-Stärke-Polysaccharid.

9. Verfahren zur Herstellung einer emulgierenden Mischung umfassend die Schritte
Mahlen von Getreide
Abtrennen einer proteinreichen Mehlfraktion
Granulieren der Mehlfraktion mit Wasser, welches 0,1 bis 2 Gew.-% Säure enthält, zu einem Mehlextraktgranulat
Dispergieren des Mehlxtraktgranulates in Wasser
Erhitzen der wässrigen Mehlextraktgranulatdispersion auf 60 bis 90 °C für 1 bis 100 Minuten sofern bei der Granulierung nicht Temepraturen von mindestens 80 °C erreicht wurden.

10. Verfahren gemäß Anspruch 9, **dadurch gekennzeichnet, dass** beim Granulieren mindestens ein Nichtgetreideprotein und/oder mindestens ein Nicht-Stärke-Polysaccharid zugesetzt werden.

11. Verfahren gemäß Anspruch 9, **dadurch gekennzeichnet, dass** der wässrigen Dispersion, ggfs. vor oder nach dem Erhitzen, mindestens ein Nichtgetreide-protein und/oder mindestens ein Nicht-Stärke-Polysaccharid zugefügt werden.

12. Verfahren gemäß einem der Ansprüche 9 bis 11, **dadurch gekennzeichnet, dass** für die Granulierung die Mehlfraktiion in Wasser suspendiert und mit einem Walzentrockner getrocknet wird.
